# EUROPEAN PATENT APPLICATION

(11) **EP 4 036 074 A1**
(43) Date of publication of application: **03.08.2022**
(21) Application number: 21153633.9
(22) Date of filing: 27.01.2021
(51) Int. Cl.: C07C 7/20, C07C 15/46

(54) **METHOD FOR SUPPRESSING THE POLYMERIZATION OF UNSATURATED AROMATIC MONOMERS**

(71) Applicant: Evonik Operations GmbH, 45128 Essen (DE)
(72) Inventor: JAMES, Phillip, SA70 7LE Tenby (GB); ERPELDINGER, Oliver, 42489 Wülfrath (DE); KERL, Thomas, 50933 Köln (DE); SCHEIPERS, Ina, 48734 Reken (DE); THUM, Oliver, 40880 Ratingen (DE)
(74) Representative: Evonik Patent Association

(57) **Abstract**

The present invention relates to a method for suppressing the polymerization of an unsaturated aromatic monomer such as styrene, comprising addition of a mixture M₁ comprising
(i) at least one of p-benzoquinone dioxime (abbreviated as "BDO"), p-benzoquinone diimide (abbreviated as "BDI") and derivatives thereof,
(ii) at least one of 2,2,6,6-tetramethylpiperidine-*N*-oxyl (abbreviated as "TEMPO") and derivatives thereof.

The present invention further relates to a mixture M₂ comprising
(i) at least one of BDO, BDI and derivatives thereof,
(ii) at least one of TEMPO and derivatives thereof,
(iii) at least one unsaturated aromatic monomer, in particular styrene.

It was surprisingly found that TEMPO and TEMPO derivatives can synergistically enhance the inhibiting effect of BDO, BDI and their derivatives on the polymerization of unsaturated aromatic monomers.

## Description

The present invention relates to a method for suppressing polymerization of an unsaturated aromatic monomer such as styrene, comprising addition of a mixture **M₁** comprising
(i) at least one of p-benzoquinone dioxime (abbreviated as "BDO"), p-benzoquinone diimide (abbreviated as "BDI") and derivatives thereof,
(ii) at least one of 2,2,6,6-tetramethylpiperidine-*N*-oxyl (abbreviated as "TEMPO") and derivatives thereof.

The present invention further relates to a mixture **M₂** comprising
(i) at least one of BDO, BDI and derivatives thereof,
(ii) at least one of TEMPO and derivatives thereof,
(iii) at least one unsaturated aromatic monomer, in particular styrene.

It was surprisingly found that TEMPO and TEMPO derivatives can synergistically enhance the inhibiting effect of BDO, BDI and their derivatives on the polymerization of unsaturated aromatic monomers.

### 1. Background Art

Unsaturated aromatic compounds such as styrene and α-methylstyrene tend to readily polymerize during production, refining and storage due to heating, light, etc.

The unsaturated aromatic monomers may be exposed to high temperatures during production and refinement. In such a case, there may be problems in that the unsaturated aromatic monomers undergo polymerization which results in lowering the yield of unsaturated aromatic monomers, and moreover, they adhere to a used device as dirt during the production or refinement process, etc., which in the ultimate can lead to a significant decrease in the production efficiency.

Conventionally, a method for adding nitrophenols to an unsaturated aromatic monomer as a polymerization inhibitor of the unsaturated aromatic monomer has been proposed in order to prevent the problems and this has actually been used in practice. Mostly, nitrophenols such as 2,4-dinitrophenol (abbreviated as "DNP") or 2,4-dinitro-6-sec-butylphenol (abbreviated as "DNBP") has often been used as a polymerization inhibitor of an unsaturated aromatic monomer.

For example, EP 1 389 607 A1 proposes a method for suppressing the polymerization of an aromatic vinyl compound which is characterized by adding 2-nitrophenols such as DNBP and DNP, and sulfonic acid compounds to an aromatic vinyl compound.

In addition, WO 2013/108266 A1 describes an additive composition for suppressing and inhibiting a polymerization of aromatic vinyl monomers including styrene, which comprises at least one aromatic nitro compound such as DNBP and at least one aliphatic tertiary amine. However, nitrophenols are highly toxic and, for example, DNP and DNBP are designated as a medicinal toxic substance by the Poisonous and Deleterious Substances Control Act. For this reason, there has been a problem in that advanced safety and preventive measures are required when using nitrophenols as a polymerization inhibitor for an unsaturated aromatic monomer.

The method described in EP 2 055 691 A1 tackles this problem and presents a method for inhibiting the polymerization of unsaturated monomers such as styrene in which a quinone methide is added to the monomer. This retarder has the advantage of reduced toxicity compared to DNBP and DNP.

A. F. Gogotov, M. V. Parilova, and A. K. Khaliullin, Russian Journal of Applied Chemistry, Vol. 75, No. 5, 2002, 811-813 describe the use of BDO to inhibit styrene polymerization.

Recently, JP 2020-111533 A presented a method for suppressing the polymerization of unsaturated aromatic monomers such as styrene by addition of a liquid mixture including BDO, BDI or derivatives thereof and at least one solvent selected from among an alcohol-based compound, an amide-based compound and an aromatic compound.

### 2. Short Description of the Invention

The present invention is made in view of the above circumstances, and an objective thereof is to provide an improved method for suppressing the polymerization of unsaturated aromatic monomers which has a low impact on the human body. Moreover, an objective thereof is to provide a method which exhibits an excellent polymerization suppression effect with a lot smaller added amount of a polymerization inhibitor.

The present invention is based on the finding that polymerization of unsaturated aromatic monomers can be suppressed with a lot smaller added amount of a BDO/ BDI-based polymerization inhibitor, i.e. at least one compound (A) represented by the formula **(I)** while reducing the impact on the human body, by adding a mixture of at least one compound (A) and at least one compound (B) represented by the formula **(II)** to an unsaturated aromatic monomer.

According to the present invention, a method for suppressing a polymerization of unsaturated aromatic monomers which has a low impact on the human body can be provided. In addition, with the use of the method for suppressing a polymerization of unsaturated aromatic monomers of the present invention, an excellent polymerization suppression effect can be exhibited with a smaller added amount of a polymerization inhibitor, which hence can lower the environmental load (NOx emission and total nitrogen emission to wastewater) and contributes to cost reduction.

### 3. Figure

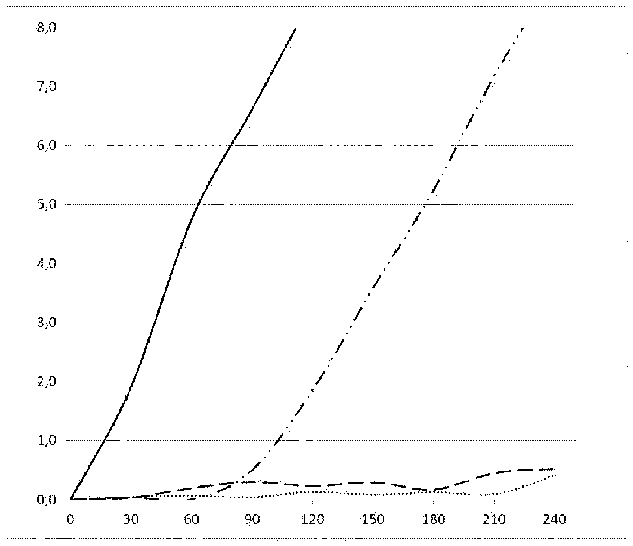

The Figure shows the results of the polymerization tests carried out without additive (Comparative Example **C1**; continuous graph), 100 ppm 4-hydroxy-2,2,6,6-tetramethylpiperidine-*N*-oxyl ("4-HT"; Comparative Example **C2**; graph "-●●-●●"), 100 ppm p-benzoquinone-dioxime ("BDO"; Comparative Example **C3**; graph "-----"), and a mixture of 50 ppm 4-HT and 50 ppm BDO (Inventive Example **I1;** graph "......."). The x-axis shows the time in minutes. The y-axis gives the relative content of polystyrene, i.e. the percentage (mass-%) of styrene that has undergone polymerization in relation to the mass of unpolymerized styrene that was initially used.

### 4. Detailed Description of the Invention

### 4.1 First Aspect: Method

In a first aspect, the present invention relates to a method for suppressing the polymerization of unsaturated aromatic monomers, which method comprises adding to an unsaturated aromatic monomer, a mixture **M₁** comprising (i) at least one compound (A) represented by the following formula **(I)** and (ii) at least one compound (B) represented by the following formula **(II)**:

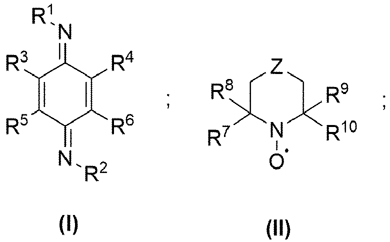

In formula **(I),** R¹ and R² are each independently selected from the group consisting of hydrogen, hydroxy, alkyl, aryl, alkaryl, aralkyl, preferably selected from the group consisting of hydroxy, alkyl, aryl, alkaryl, aralkyl, more preferably selected from the group consisting of hydroxy, alkyl, aryl, most preferably both hydroxy.

In formula **(I),** R³, R⁴, R⁵, R⁶ are each independently selected from the group consisting of hydrogen, hydroxy, alkyl, aryl, alkaryl, aralkyl, preferably selected from the group consisting of hydrogen, alkyl, aryl, more preferably R³, R⁴, R⁵, R⁶ are all hydrogen.

In a preferred embodiment, the compound (A) represented by the abovementioned formula **(I)** is selected from
- *N*,*N*'-dialkyl-*p*-benzoquinonediimide, which is preferably selected from *N*,*N*'-di-*sec*-butyl-*p*-benzoquinonediimide and *N*,*N*'-bis(1,4-dimethylpentyl)-p-benzoquinonediimide;
- *N*-phenyl-*N*'-alkyl-*p*-benzoquinone, which is preferably selected from *N*-phenyl-*N*'-methyl-*p*-benzoquinonediimide, *N*-phenyl-*N*'-ethyl-p-benzoquinonediimide, *N*-phenyl-*N*'-propyl-*p*-benzoquinonediimide, *N*-phenyl-*N*'-*n*-butyl-p-benzoquinonediimide, *N*-phenyl-*N*'-iso-butyl-*p*-benzoquinonediimide, *N*-phenyl-*N*'-*sec*-butyl-p-benzoquinonediimide, *N*-phenyl-*N*'-tert-butyl-*p*-benzoquinonediimide, *N*-phenyl-*N*'-*n*-pentyl-*p*-benzoquinonediimide, *N*-phenyl-*N*'-(1-methylhexyl)-*p*-benzoquinonediimide, *N*-phenyl-*N*'-(1,3-dimethylhexyl)-p-benzoquinonediimide, *N*-phenyl-*N*'-(1,4-dimethylpentyl)-p-benzoquinonediimide, *N*-phenyl-*N*'-(1,4-dimethylbutyl)-*p*-benzoquinonediimide;
- *N*,*N*'-(cyclohexa-2,5-dien-1,4-diylidene)dibutane-1-amine; (E)-N-[(E)-4-(4-methylpentan-2-ylimino)cyclohexa-2,5-dienylidene]aniline;
- *p*-benzoquinone dioxime.

These may be used in singly or in combination of two or more kinds.

From among these, p-benzoquinone dioxime is preferable as compound (A) from the viewpoint of obtaining an excellent polymerization suppression effect.

In formula **(II),** R⁷, R⁸, R⁹, R¹⁰ are each independently selected from alkyl having 1 to 4 carbon atoms, more preferably all are methyl.

In formula **(II),** Z is selected from the group consisting of >CR¹¹R¹², >C=O, >CH-OH, >CH-NR¹³R¹⁴, >CH-Hal, >CH-OR¹⁵, >CH-COOR¹⁶, >CH-O-CO-NHR¹⁷, >CH-NH-CO-R¹⁸, 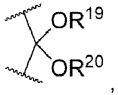 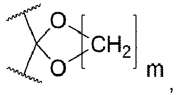
preferably Z is selected from the group consisting of >CR¹¹R¹², >C=O, >CH-NR¹³R¹⁴, >CH-Hal, >CH-OR¹⁵, >CH-COOR¹⁶, >CH-O-CO-NHR¹⁷, >CH-NH-CO-R¹⁸,
   more preferably Z is selected from the group consisting of >C=O, >CH-NR¹³R¹⁴, >CH-OR¹⁵, >CH-NH-CO-R18,
   most preferably, Z is >CH-OH,
wherein R¹¹, R¹², R¹³, R¹⁴, R¹⁵, R¹⁶, R¹⁷, R¹⁸, R¹⁹, R²⁰ are each independently selected from the group consisting of hydrogen, alkyl group having 1 to 6 carbon atoms,
   Hal is selected from the group consisting of fluorine, chlorine, bromine or iodine, preferably chlorine, bromine, most preferably chlorine,
   m = 1 to 4.

For clarification, it is pointed out, that:
- when Z is >CR¹¹R¹², formula **(II)** has the following structure **(II-A),**
- when Z is >C=O, formula **(II)** has the following structure **(II-B),**
- when Z is >CH-NR¹³R¹⁴, formula **(II)** has the following structure **(II-C),**
- when Z is >CH-Hal, formula **(II)** has the following structure **(II-D),**
- when Z is >CH-OR¹⁵, formula **(II)** has the following structure **(II-E),**
- when Z is >CH-COOR¹⁶, formula **(II)** has the following structure **(II-F),**
- when Z is >CH-O-CO-NHR¹⁷, formula **(II)** has the following structure **(II-G),**
- when Z is >CH-NH-CO-R¹⁸, formula **(II)** has the following structure **(II-H),**
- when Z is 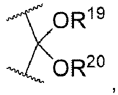 formula **(II)** has the following structure **(II-I)**,
- when Z is > 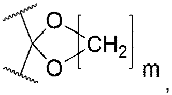 formula **(II)** has the following structure **(II-J):**

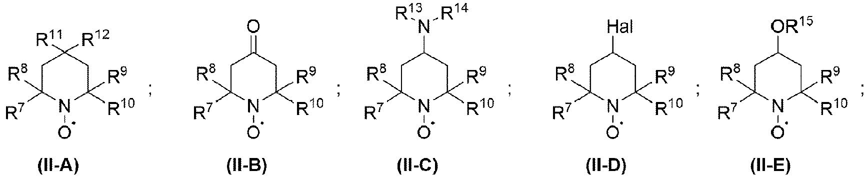

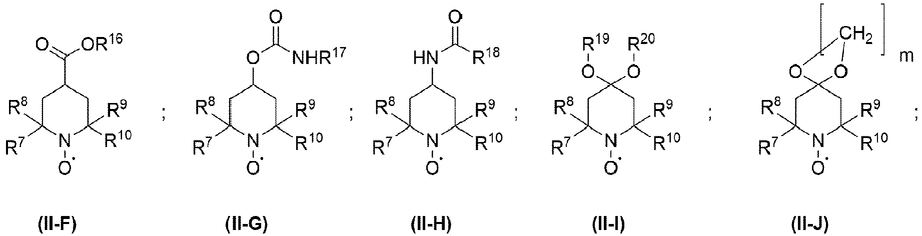

According to the invention, an alkyl group preferably has 1 to 10 carbon atoms, more preferably 1 to 6 carbon atoms, even more preferably 1 to 4 carbon atoms, even more preferably is selected from methyl, ethyl, *n*-propyl, *iso*-propyl, even more preferably is selected from methyl, ethyl.

According to the invention, an aryl group preferably has 1 to 10 carbon atoms, is more preferably selected from phenyl, naphthyl, and even more preferably phenyl.

According to the invention, an alkaryl group preferably has 1 to 15 carbon atoms, is more preferably selected from tolyl, ethylphenyl, cumyl, dimethylphenyl, diethylphenyl, di-*iso*-propylphenyl, trimethylphenyl, triethylphenyl, tri-*iso*-propylphenyl, and is even more preferably phenyl.

According to the invention, an aralkyl group preferably has 1 to 15 carbon atoms, is more preferably selected from methylbenzyl, ethylbenzyl, *iso*-propylbenzyl, and is even more preferably benzyl.

Even more preferably, the compound (B) is selected from the group consisting of
2,2,6,6-tetramethylpiperidine-*N*-oxyl,
4-acetamido-2,2,6,6-tetramethylpiperidine-*N*-oxyl,
4-hydroxy-2,2,6,6-tetramethylpiperidine-*N*-oxyl,
4-oxo-2,2,6,6-tetramethylpiperidine-*N*-oxyl,
a compound of the structure **(II)** where R⁷ to R¹⁰ are each methyl and Z is >CH-OR¹⁵ where R¹⁵ = alkyl group having 1 to 6 carbon atoms,
a compound of the structure **(II)** where R⁷ to R¹⁰ are each a methyl group and Z is

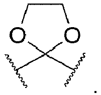

Even more preferably, the compound (B) is selected from the group consisting of
2,2,6,6-tetramethylpiperidine-*N*-oxyl,
4-hydroxy-2,2,6,6-tetramethylpiperidine-*N*-oxyl,
4-oxo-2,2,6,6-tetramethylpiperidine-*N*-oxyl.

Most preferably, the compound (B) is 4-hydroxy-2,2,6,6-tetramethylpiperidine-*N*-oxyl (abbreviated as "4-HT").

According to the method for suppressing the polymerization of an unsaturated aromatic monomer, comprising adding, to an unsaturated aromatic monomer, a mixture M₁ comprising (i) at least one compound (A) represented by formula **(I)** and (ii) at least one compound (B) represented by the following formula **(II),** an excellent polymerization suppression effect is exhibited with a lot smaller added amount of a polymerization inhibitor while impact on the human body is low. Herein, in view of impact on the human body, it is preferable not to add DNP or DNBP.

In a preferred embodiment, the mixture **M₁** also includes a solvent, so the mixture **M₁** is liquid. The solvent is preferably selected from aliphatic hydrocarbons, aromatic compounds, alcohols, amides, water.

More preferably, the solvent is selected from aliphatic hydrocarbons with 6 to 15 carbon atoms, aromatic compounds with 6 to 15 carbon atoms, alcohols with 1 to 6 carbon atoms, amides, water, more preferably selected from hexane, cyclohexane, styrene, benzene, toluene, xylene, styrene, ethylbenzene, phenol, methanol, ethanol, *n*-propanol, *iso*-propanol, *n*-butanol, *n*-hexanol, *N*,*N*-dimethylisobutylamide, *N*,*N*-dimethylformamide, *N*,*N*-dimethylacetamide, *N*-methyl-2-pyrrolidone, *N*-methylcaprolactam, 1,3-dimethylimidazolidinone, tetramethylurea, water,
more preferably selected from benzene, phenol, toluene, ethylbenzene, xylene, styrene, methanol, *N*,*N*-dimethylformamide, *N*-methyl-2-pyrrolidone.

The unsaturated aromatic monomer is preferably selected from the group consisting of
- styrene;
- divinylbenzene;
- alkyl styrene, preferably selected from the group consisting of α-methylstyrene, *p*-methylstyrene, *p*-ethylstyrene, *p*-*n*-propylstyrene, *p*-*iso*-propylstyrene, *p*-*n*-butylstyrene, *p*-*tert*-butylstyrene, *p*-phenylstyrene, *o*-methylstyrene, *o*-ethylstyrene, *o*-*n*-propylstyrene, *o*-*iso*-propylstyrene, *m*-methylstyrene, *m*-ethylstyrene, *m*-*n*-propylstyrene, *m*-*iso*-propylstyrene, *m*-*n*-butylstyrene, mesityl styrene, 2,4-dimethylstyrene, 2,5-dimethylstyrene, 3,5-dimethylstyrene, 3-vinylstyrene, 4-vinylstyrene, 4-butenylstyrene;
- halogenated styrene, preferably selected from the group consisting of *p*-chlorostyrene, *m*-chlorostyrene, *o*-chlorostyrene, *p*-bromostyrene, *m*-bromostyrene, *o*-bromostyrene, *p*-fluorostyrene, *m*-fluorostyrene, *o*-fluorostyrene, *o*-methyl-p-fluorostyrene;
- alkoxy styrene, preferably selected from the group consisting of p-methoxystyrene, *o*-methoxystyrene, *m*-methoxystyrene;
- vinylbenzoic acid ester. These unsaturated aromatic monomers may be used singly or in combination of two or more kinds.

As unsaturated aromatic monomer, styrene, divinylbenzene and α-methylstyrene are preferable, and styrene is most preferable.

The added amount of the compounds (A) and (B) can be handled by the skilled person according to need.

It is preferable that in the method according to the first aspect of the invention, the mixture **M₁** is added in such a way that the total amount of all added compounds (A) is from 0.1 to 10000 mass ppm, more preferably from 1 to 1000 mass ppm, even more preferably from 10 to 500 mass ppm, further preferably from 20 to 400 mass ppm, even further preferably from 30 to 300 mass ppm, more preferably 40 to 100, even more preferably 50 to 80 ppm, most preferably 50 ppm with respect to the total mass of all unsaturated aromatic monomers.

It is preferable that in the method according to the first aspect of the invention, the mixture **M₁** is added in such a way that the total amount of all added compounds (B) is from 0.1 to 10000 mass ppm, more preferably from 1 to 1000 mass ppm, even more preferably from 10 to 500 mass ppm, further preferably from 20 to 400 mass ppm, even further preferably from 30 to 300 mass ppm, more preferably 40 to 100, even more preferably 50 to 80 ppm, most preferably 50 ppm with respect to the total mass of all unsaturated aromatic monomers.

In another embodiment, it is advantageous, as the compound (B) can be used to boost the inhibitory effect of compound (A), to use compound (A) in excess with respect to compound (B). In this embodiment, it is preferably that in the method according to the first aspect of the invention, the mixture **M₁** is added in such a way that the total amount of all added compounds (A) is from 0.1 to 10000 mass ppm, more preferably from 1 to 1000 mass ppm, even more preferably from 10 to 500 mass ppm, further preferably from 20 to 400 mass ppm, even further preferably from 30 to 300 mass ppm, more preferably 40 to 100, even more preferably 50 to 80 ppm, most preferably 50 ppm with respect to the total mass of the unsaturated aromatic monomer and that the mass weight total amount of all added compounds (B) is from 0.01 to 100 weight-% of the mass weight total amount of all added compounds (A), preferably from 0.1 to 80 weight-%, more preferably from 1 to 50 weight-%, even more preferably from 1 to 10 weight-% of the mass weight total amount of all added compounds (A).

When the mixture **M₁** contains a solvent, the total content of the compounds (A) and (B) in the liquid mixture **M₁** is preferably from 0.001 to 50 mass%, more preferably 0.01 to 30 mass%.

According to the invention, when contemplating the use of the mixture **M₁** in a technical or industrial context, there is no particular restriction concerning the spot for adding the liquid mixture to the unsaturated aromatic monomer in the present invention, as long as the mixture **M₁** after addition suppresses the polymerization of an unsaturated aromatic monomer in the production plant. When a styrene production plant or another plant in which an unsaturated aromatic monomer are produced or handled is contemplated, such addition can be made before a splitter in which styrene produced and unreacted ethylbenzene are separated by means of distillation and it is possible to carry out the addition at the bottom lines of splitters.
The place for adding the preferably liquid mixture **M₁** to the unsaturated aromatic monomer according to the invention may be, for example, a distillation tower of unsaturated aromatic monomers, places where polymerization reactions occur such as the top and bottom of a reaction tower and a storage tank, places where fouling (dirt) occurs, or any upstream stage thereto. The upstream stage may be, for example, a distillation tower group, etc., which is immediately after an ethylbenzene dehydrogenation reaction in case of styrene, since styrene is generally produced by a dehydrogenation reaction of ethylbenzene and the produced styrene and unreacted ethylbenzene are continuously subjected to separation by distillation for purification.

The place for adding the preferably liquid mixture **M₁** to the unsaturated aromatic monomer according to the invention is preferably a distillation stage, and more preferably a distillation tower group that is immediately after the dehydrogenation reaction of ethylbenzene.

The addition method may be a method in which the mixture is added at once to the specific place, or a method in which the mixture is separately added to multiple places, etc., and the method may be selected as appropriate. In addition, the mixture may be added continuously or intermittently.

For example, the compound (A) may be added in a different spot of the plant than compound (B), so that the two compounds mix and form mixture **M₁** in the plant.

According to the present invention, the unsaturated aromatic monomer exhibits a better effect in a system having a temperature of 110°C or higher.

### 4.2 Second Aspect: Mixture M₂

In a second aspect, the present invention relates to a mixture **M₂** for suppressing the polymerization of unsaturated aromatic monomers,
(i) at least one compound (A) represented by the following formula **(I),**
(ii) at least one compound (B) represented by the following formula **(II),**
(iii) at least one unsaturated aromatic monomer,
wherein 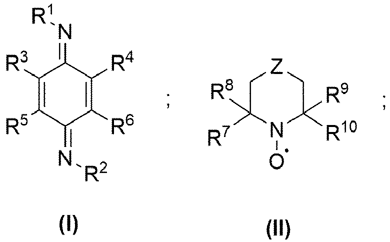 wherein in formula **(I),** R¹ and R² are each independently selected from the group consisting of hydrogen, hydroxy, alkyl, aryl, alkaryl, aralkyl, preferably selected from the group consisting of hydroxy, alkyl, aryl, alkaryl, aralkyl, more preferably selected from the group consisting of hydroxy, alkyl, aryl, most preferably both hydroxy.

In formula **(I)**, R³, R⁴, R⁵, R⁶ are each independently selected from the group consisting of hydrogen, hydroxy, alkyl, aryl, alkaryl, aralkyl, preferably selected from the group consisting of hydrogen, alkyl, aryl, more preferably R³, R⁴, R⁵, R⁶ are all hydrogen.

In a preferred embodiment, the compound (A) represented by the abovementioned formula **(I)** is selected from
- *N*,*N*'-dialkyl-*p*-benzoquinonediimide, which is preferably selected from *N*,*N*'-di-*sec*-butyl-*p*-benzoquinonediimide and *N*,*N*'-bis(1,4-dimethylpentyl)-p-benzoquinonediimide;
- *N*-phenyl-*N*'-alkyl-*p*-benzoquinone, which is preferably selected from *N*-phenyl-*N*'-methyl-*p*-benzoquinonediimide, *N*-phenyl-*N*'-ethyl-p-benzoquinonediimide, *N*-phenyl-*N*'-propyl-*p*-benzoquinonediimide, *N*-phenyl-*N*'-*n*-butyl-*p*-benzoquinonediimide, *N*-phenyl-*N*'-*iso*-butyl-*p*-benzoquinonediimide, *N*-phenyl-*N*'-*sec*-butyl-p-benzoquinonediimide, *N*-phenyl-*N*'-*tert*-butyl-*p*-benzoquinonediimide, *N*-phenyl-*N*'-*n*-pentyl-*p*-benzoquinonediimide, *N*-phenyl-*N*'-(1-methylhexyl)-*p*-benzoquinonediimide, *N*-phenyl-*N*'-(1,3-dimethylhexyl)-*p*-benzoquinonediimide, *N*-phenyl-*N*'-(1,4-dimethylpentyl)-p-benzoquinonediimide, *N*-phenyl-*N*'-(1,4-dimethylbutyl)-*p*-benzoquinonediimide;
- *N*,*N*'-(cyclohexa-2,5-dien-1,4-diylidene)dibutane-1-amine; (E)-N-[(E)-4-(4-methylpentan-2-ylimino)cyclohexa-2,5-dienylidene]aniline;
- *p*-benzoquinone dioxime.

These may be used in singly or in combination of two or more kinds.

From among these, p-benzoquinone dioxime is preferable as compound (A) from the viewpoint of obtaining an excellent polymerization suppression effect.

In formula **(II),** R⁷, R⁸, R⁹, R¹⁰ are each independently selected from alkyl having 1 to 4 carbon atoms, more preferably all are methyl.

Z is selected from the group consisting of >CR¹¹R¹², >C=O, >CH-NR¹³R¹⁴, >CH-Hal, >CH-OR¹⁵, >CH-COOR¹⁶, >CH-O-CO-NHR¹⁷, >CH-NH-CO-R¹⁸, 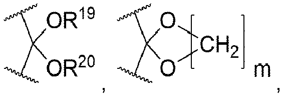 wherein R¹¹, R¹², R¹³, R¹⁴, R¹⁵, R¹⁶, R¹⁷, R¹⁸, R¹⁹, R²⁰ are each independently selected from the group consisting of hydrogen, alkyl group having 1 to 6 carbon atoms,
Hal is selected from the group consisting of fluorine, chlorine, bromine or iodine,
m = 1 to 4.

In formula **(II)**, Z is selected from the group consisting of >CR¹¹R¹², >C=O, >CH-OH, >CH-NR¹³R¹⁴, >CH-Hal, >CH-OR¹⁵, >CH-COOR¹⁶, >CH-O-CO-NHR¹⁷, >CH-NH-CO-R¹⁸, 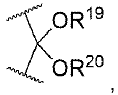 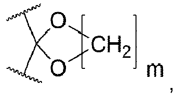
preferably Z is selected from the group consisting of >CR¹¹R¹², >C=O, >CH-NR¹³R¹⁴, >CH-Hal, >CH-OR¹⁵, >CH-COOR¹⁶, >CH-O-CO-NHR¹⁷, >CH-NH-CO-R¹⁸,
   more preferably Z is selected from the group consisting of >C=O, >CH-NR¹³R¹⁴, >CH-OR¹⁵, >CH-NH-CO-R¹⁸,
   most preferably, Z is >CH-OH,

wherein R¹¹, R¹², R¹³, R¹⁴, R¹⁵, R¹⁶, R¹⁷, R¹⁸, R¹⁹, R²⁰ are each independently selected from the group consisting of hydrogen, alkyl group having 1 to 6 carbon atoms,
   Hal is selected from the group consisting of fluorine, chlorine, bromine or iodine, preferably chlorine, bromine, most preferably chlorine,
   m = 1 to 4.

Even more preferably, the compound (B) is selected from the group consisting of
2,2,6,6-tetramethylpiperidine-*N*-oxyl,
4-acetamido-2,2,6,6-tetramethylpiperidine-*N*-oxyl,
4-hydroxy-2,2,6,6-tetramethylpiperidine-*N*-oxyl,
4-oxo-2,2,6,6-tetramethylpiperidine-*N*-oxyl,
a compound of the structure **(II)** where R⁷ to R¹⁰ are each methyl and Z is >CH-OR¹⁵ where R¹⁵ = alkyl group having 1 to 6 carbon atoms, a compound of the structure **(II)** where R⁷ to R¹⁰ are each a methyl group and Z is

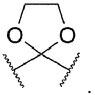

Even more preferably, the compound (B) is selected from the group consisting of
2,2,6,6-tetramethylpiperidine-*N*-oxyl,
4-hydroxy-2,2,6,6-tetramethylpiperidine-*N*-oxyl,
4-oxo-2,2,6,6-tetramethylpiperidine-*N*-oxyl.

Most preferably, the compound (B) is 4-hydroxy-2,2,6,6-tetramethylpiperidine-*N*-oxyl (abbreviated as "4-HT").

The unsaturated aromatic monomer is preferably selected from the group consisting of
- styrene;
- divinylbenzene;
- alkyl styrene, preferably selected from the group consisting of α-methylstyrene, *p*-methylstyrene, *p*-ethylstyrene, *p-n*-propylstyrene, *p*-*iso*-propylstyrene, *p-n*-butylstyrene, *p*-*tert*-butylstyrene, *p*-phenylstyrene, *o*-methylstyrene, *o*-ethylstyrene, *o*-*n*-propylstyrene, *o*-*iso*-propylstyrene, *m*-methylstyrene, *m*-ethylstyrene, *m*-*n*-propylstyrene, *m*-*iso*-propylstyrene, *m*-*n*-butylstyrene, mesityl styrene, 2,4-dimethylstyrene, 2,5-dimethylstyrene, 3,5-dimethylstyrene, 3-vinylstyrene, 4-vinylstyrene, 4-butenylstyrene;
- halogenated styrene, preferably selected from the group consisting of p-chlorostyrene, *m*-chlorostyrene, *o*-chlorostyrene, *p*-bromostyrene, *m*-bromostyrene, *o-*bromostyrene, *p*-fluorostyrene, *m*-fluorostyrene, *o*-fluorostyrene, *o*-methyl-*p*-fluorostyrene;
- alkoxy styrene, preferably selected from the group consisting of *p*-methoxystyrene, *o*-methoxystyrene, *m*-methoxystyrene;
- vinylbenzoic acid ester. These unsaturated aromatic monomers may be used singly or in combination of two or more kinds.

As unsaturated aromatic monomer, styrene, divinylbenzene and α-methylstyrene are preferable, and styrene is most preferable.

The mixture **M₂** for suppressing the polymerization of an unsaturated aromatic monomer, displays an excellent polymerization suppression effect with a lot smaller added amount of a polymerization inhibitor while impact on the human body is low. Herein, in view of impact on the human body, it is preferable not to add DNP or DNBP.

The mixture **M₂** is preferably liquid.

In a preferred embodiment, the mixture **M₂** also includes a solvent. The solvent is preferably selected from aliphatic hydrocarbons, aromatic compounds, alcohols, amides, water,

More preferably selected from aliphatic hydrocarbons with 6 to 15 carbon atoms, aromatic compounds with 6 to 15 carbon atoms, alcohols with 1 to 6 carbon atoms, amides, water,
more preferably selected from hexane, cyclohexane, styrene, benzene, toluene, xylene, styrene, ethylbenzene, phenol, methanol, ethanol, *n*-propanol, *iso*-propanol, *n*-butanol, *n*-hexanol, *N*,*N*-dimethylisobutylamide, *N*,*N*-dimethylformamide, *N*,*N* -dimethylacetamide, *N*-methyl-2-pyrrolidone, *N*-methylcaprolactam, 1,3-dimethylimidazolidinone, tetramethylurea, water,
more preferably selected from benzene, phenol, toluene, ethylbenzene, xylene, styrene, methanol, *N*,*N*-dimethylformamide, *N*-methyl-2-pyrrolidone.

The amounts of the compounds (A) and (B) in mixture **M₂** can be handled by the skilled person according to need.

It is preferable that the total amount of all compounds (A) comprised by mixture **M₂** is from 0.1 to 10000 mass ppm, more preferably from 1 to 1000 mass ppm, even more preferably from 10 to 500 mass ppm, further preferably from 20 to 400 mass ppm, even further preferably from 30 to 300 mass ppm, more preferably 40 to 100, even more preferably 50 to 80 ppm, most preferably 50 ppm with respect to the total mass of the unsaturated aromatic monomer comprised by mixture **M₂.**

It is preferable that the total amount of all added compounds (B) comprised by mixture **M₂** is from 0.1 to 10000 mass ppm, more preferably from 1 to 1000 mass ppm, even more preferably from 10 to 500 mass ppm, further preferably from 20 to 400 mass ppm, even further preferably from 30 to 300 mass ppm, more preferably 40 to 100, even more preferably 50 to 80 ppm, most preferably 50 ppm with respect to the total mass of the unsaturated aromatic monomer comprised by mixture **M₂.**

In another embodiment, it is advantageous, as the compound (B) can be used to boost the inhibitory effect of compound (A), that the mixture **M₂** comprises compound (A) in excess with respect to compound (B). In this embodiment, it is preferably that the total amount of all compounds (A) comprised by mixture **M₂** is from 0.1 to 10000 mass ppm, more preferably from 1 to 1000 mass ppm, even more preferably from 10 to 500 mass ppm, further preferably from 20 to 400 mass ppm, even further preferably from 30 to 300 mass ppm, more preferably 40 to 100, even more preferably 50 to 80 ppm, most preferably 50 ppm with respect to the total mass of the unsaturated aromatic monomer comprised by mixture **M₂** and that the mass weight total amount of all compounds (B) comprised by mixture **M₂** is from 0.01 to 100 weight-% of the mass weight total amount of all added compounds (A), preferably from 0.1 to 80 weight-%, more preferably from 1 to 50 weight-%, even more preferably from 1 to 10 weight-% of the mass weight total amount of all compounds (A) comprised by mixture **M₂.**

When the mixture **M₂** contains a solvent, the total content of the compounds (A) and (B) in the mixture **M₂** is preferably from 0.001 to 50 mass%, more preferably 0.01 to 30 mass%.

### Examples

### Examples C1, C2, C3, I1

Commercially available, stabilized styrene was freed of the *tert*-butyl-1,2-hydroxybenzene (TBC) stabilizer at a reduced pressure of 100 hPa and a bottom temperature of 75°C in an inert nitrogen atmosphere.

The test apparatus, which consisted of a four-neck flask equipped with a thermometer, a reflux condenser, a septum and a precision glass stirrer, was purged thoroughly with nitrogen in order to obtain an oxygen-free atmosphere. 300 g of the unstabilized styrene were added to the three-neck flask and admixed with 100 ppm of an additive as follows:
Comparative Example **C1:** blind (no additive).
Comparative Example **C2:** 100 ppm 4-HT.
Comparative Example **C3:** 100 ppm p-benzoquinone-dioxime.
Inventive Example **I1:** 50 ppm 4-HT, 50 ppm p-benzoquinone-dioxime.
The constant nitrogen supply through a glass frit into the styrene solution enabled an inert nitrogen atmosphere over the entire test period. The styrene solution was stirred vigorously. At the start of the experiment, the flask was immersed into an oil bath preheated to 110 °C to such an extent that the stabilized styrene solution was completely immersed. After the immersion of the three-neck flask into the heated oil bath, 3 g of the styrene solution were withdrawn at regular intervals via the septum, weighed accurately and added to 50 ml of methanol. The methanol mixture was stirred at room temperature for half an hour. The methanol brought about the precipitation of the polystyrene formed during the test. This was removed by filtration through a glass filter crucible. The filter residue was washed with 20 ml of methanol and then dried at 110 °C for at least 5 hours. The polystyrene remaining in the glass filter crucible was then weighed. The value determined and the initial weight were used to determine the percentage of polymer. This polymer content, i.e. the percentage (mass-%) of styrene that has undergone polymerization in relation to the mass of unpolymerized styrene that was initially used (y-axis) was plotted against the reaction time (x-axis) in the Figure. The results are shown in the following table.

As can be seen from the table, the results of which are summarized in the Figure, the addition of 4-HT (**C2**) provides a longer time period compared to the result according to **C1** for formation of the same amount of polymer, which proves the inhibitory action of 4-HT on styrene polymerization. *p*-Benzoquinone-dioxime also shows inhibitory action, as can be seen from the comparison of **C3** and **C1**.
Surprisingly, when both 4-HT and *p*-benzoquinone-dioxime are used in combination, the inhibitory action is not only lower, but also pertains for a longer period of time. This can be seen when comparing the curves in **C2** and **C3** with the curve according to **I1**. While **C2** shows that after a certain amount of time (∼ 210 minutes), the inhibitory action of 4-HT alone vanishes, the enhanced inhibitory action of the combination of 4-HT and p-benzoquinone (confer **I1** and **C3**) is maintained. This shows that there is a synergistic inhibitory effect in the combined use of 4-HT and p-benzoquinone-dioxime.

| | **Polymer content (%)** | | | |
|---|---|---|---|---|
| **Example** | **C1** | **C2** | **C3** | **11** |
| **Time (minutes)** | | | | |
| 0 | 0 | | | |
| 30 | 1.90 | 0.04 | 0.04 | 0.05 |
| 60 | 4.75 | 0.01 | 0.20 | 0.08 |
| 90 | 6.62 | 0.50 | 0.31 | 0.05 |
| 120 | 8.52 | 1.86 | 0.24 | 0.14 |
| 150 | - | 3.59 | 0.30 | 0.09 |
| 180 | - | 5.25 | 0.18 | 0.13 |
| 210 | - | 7.19 | 0.45 | 0.10 |
| 240 | - | 8.86 | 0.52 | 0.42 |

## Claims

1. Method for suppressing the polymerization of an unsaturated aromatic monomer, comprising adding to an unsaturated aromatic monomer, a mixture **M₁** comprising
(i) at least one compound (A) represented by the following formula **(I)** and
(ii) at least one compound (B) represented by the following formula **(II):** 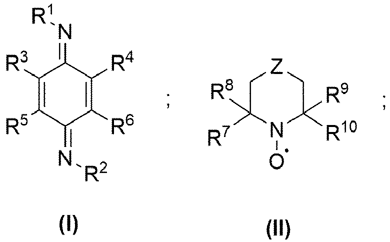
wherein in formula (I), R¹ to R² are each independently selected from the group consisting of hydrogen, hydroxy, alkyl, aryl, alkaryl, aralkyl,
R³, R⁴, R⁵, R⁶ are each independently selected from the group consisting of hydrogen, hydroxy, alkyl, aryl, alkaryl, aralkyl,
wherein in formula **(II)**, R⁷, R⁸, R⁹, R¹⁰ are each independently selected from alkyl having 1 to 4 carbon atoms,
Z is selected from the group consisting of >CR¹¹R¹², >C=O, >CH-NR¹³R¹⁴, >CH-Hal, >CH-OR¹⁵, >CH-COOR¹⁶, >CH-O-CO-NHR¹⁷, >CH-NH-CO-R¹⁸, 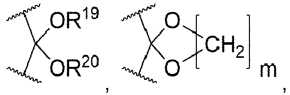 wherein R¹¹, R¹², R¹³, R¹⁴, R¹⁵, R¹⁶, R¹⁷, R¹⁸, R¹⁹, R²⁰ are each independently selected from the group consisting of hydrogen, alkyl group having 1 to 6 carbon atoms,
Hal is selected from the group consisting of fluorine, chlorine, bromine or iodine,
m = 1 to 4.

2. Method according to Claim 1, wherein in formula **(I)**,
R¹ to R² are each independently selected from the group consisting of hydroxy, alkyl, aryl, alkaryl, aralkyl,
R³, R⁴, R⁵, R⁶ are each hydrogen.

3. Method according to Claim 1 or 2, wherein the compound (B) is selected from the group consisting of
2,2,6,6-tetramethylpiperidine-*N*-oxyl,
4-acetamido-2,2,6,6-tetramethylpiperidine-*N*-oxyl,
4-hydroxy-2,2,6,6-tetramethylpiperidine-*N*-oxyl,
4-oxo-2,2,6,6-tetramethylpiperidine-*N*-oxyl,
a compound of the structure **(II)** where R⁷ to R¹⁰ are each methyl and Z is >CH-OR¹⁵ where R¹⁵ = alkyl group having 1 to 6 carbon atoms, a compound of the structure **(II)** where R⁷ to R¹⁰ are each methyl group and Z is

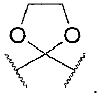

4. Method according to one of Claims 1 to 3, wherein the mixture **M₁** further comprises a solvent selected from aliphatic hydrocarbons, aromatic compounds, alcohols, amides, water.

5. Method according to one of Claims 1 to 4, wherein the unsaturated aromatic monomer is at least one selected from the group consisting of styrene, divinylbenzene, α-methylstyrene.

6. Method according to one of Claims 1 to 5, wherein the mixture **M₁** is added in such a way that the total amount of all added compounds (A) is from 0.1 to 10000 mass ppm with respect to the total mass of all unsaturated aromatic monomers and that the total amount of all added compounds (B) is from 0.1 to 10000 mass ppm with respect to the total mass of all unsaturated aromatic monomers.

7. Mixture **M₂** for suppressing the polymerization of an unsaturated aromatic monomer, comprising
(i) at least one compound (A) represented by the following formula **(I)**,
(ii) at least one compound (B) represented by the following formula **(II)**,
(iii) at least one unsaturated aromatic monomer,
wherein 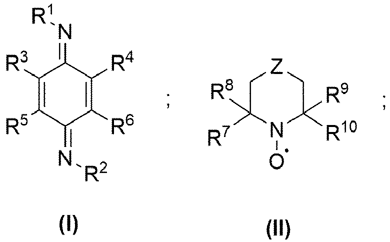
wherein in formula **(I)**, R¹ to R² are each independently selected from the group consisting of hydrogen, hydroxy, alkyl, aryl, alkaryl, aralkyl,
R³, R⁴, R⁵, R⁶ are each independently selected from the group consisting of hydrogen, hydroxy, alkyl, aryl, alkaryl, aralkyl,
wherein in formula **(II)**, R⁷, R⁸, R⁹, R¹⁰ are each independently selected from alkyl having 1 to 4 carbon atoms,
Z is selected from the group consisting of >CR¹¹R¹², >C=O, >CH-NR¹³R¹⁴, >CH-Hal, >CH-OR¹⁵, >CH-COOR¹⁶, >CH-O-CO-NHR¹⁷, >CH-NH-CO-R¹⁸, 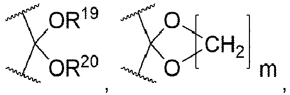 wherein R¹¹, R¹², R¹³, R¹⁴, R¹⁵, R¹⁶, R¹⁷, R¹⁸, R¹⁹, R²⁰ are each independently selected from the group consisting of hydrogen, alkyl group having 1 to 6 carbon atoms,
Hal is selected from the group consisting of fluorine, chlorine, bromine or iodine,
m = 1 to 4.

8. Mixture **M₂** according to Claim 7, wherein in formula **(I)**,
R¹ to R² are each independently selected from the group consisting of hydroxy, alkyl, aryl, alkaryl, aralkyl,
R³, R⁴, R⁵, R⁶ are each hydrogen.

9. Mixture **M₂** according to Claim 7 or 8, wherein the compound (B) is selected from the group consisting of
2,2,6,6-tetramethylpiperidine-*N*-oxyl,
4-acetamido-2,2,6,6-tetramethylpiperidine-*N*-oxyl, 4-hydroxy-2,2,6,6-tetramethylpiperidine-*N*-oxyl,
4-oxo-2,2,6,6-tetramethylpiperidine-*N*-oxyl,
a compound of the structure **(II)** where R⁷ to R¹⁰ are each methyl and Z is >CH-OR¹⁵ where R¹⁵ = alkyl group having 1 to 6 carbon atoms,
a compound of the structure **(II)** where R⁷ to R¹⁰ are each methyl group and Z is

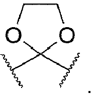

10. Mixture **M₂** according to one of Claims 7 to 9, wherein the unsaturated aromatic monomer is at least one selected from the group consisting of styrene, divinylbenzene, α-methylstyrene.

11. Mixture **M₂** according to one of Claims 7 to 10, wherein the mixture **M₂** further comprises a solvent selected from aliphatic hydrocarbons, aromatic compounds, alcohols, amides, water.

12. Mixture **M₂** according to one of Claims 7 to 11, wherein in the mixture **M₂** the total amount of all compounds (A) comprised by mixture **M₂** is from 0.1 to 10000 mass ppm with respect to the total mass of the unsaturated aromatic monomer comprised by mixture **M₂** and wherein the total amount of all added compounds (B) comprised by mixture **M₂** is from 0.1 to 10000 mass ppm with respect to the total mass of the unsaturated aromatic monomer comprised by mixture **M₂.**
